## Europäisches Patentamt
## European Patent Office
## Office européen des brevets

(19)

(11) Numéro de publication: **0 096 913**
**B1**

(12) **FASCICULE DE BREVET EUROPEEN**

(45) Date de publication du fascicule du brevet:
08.10.86

(51) Int. Cl.⁴: **C 07 D 307/36,** B 01 J 37/34

(21) Numéro de dépôt: **83200737.1**

(22) Date de dépôt: **25.05.83**

(54) Procédé amélioré de décarbonylation du furfural en vue d'obtenir du furanne.

(30) Priorité: **16.06.82 FR 8210925**

(43) Date de publication de la demande:
**28.12.83 Bulletin 83/52**

(45) Mention de la délivrance du brevet:
**08.10.86 Bulletin 86/41**

(84) Etats contractants désignés:
**AT BE CH DE GB IT LI LU NL SE**

(56) Documents cités:
**US - A - 3 007 941**

**CHEMICAL ABSTRACTS, vol. 86, no. 20, 16 mai 1977, page 427, no. 146478j, Columbus, Ohio, USA BOR-HER CHEN et al.: "The role of ultraviolet radiation in promoting the palladium-catalyzed oxidation of carbon monoxide"**
**CHEMICAL ABSTRACTS, vol. 95, no. 5, 03 août 1981, page 701, no. 42433k, Columbus, Ohio, USA K.K. KUZEMBAEV: "Effect of irradiation on low-percentage platinum and palladium catalysts on silica gel."**

**Le dossier contient des informations techniques présentées postérieurement au dépôt de la demande et ne figurant pas dans le présent fascicule.**

(73) Titulaire: **AGRIFURANE S.A., Z.I. de Boé Cidex 4412, F-47240 Bon Encontre (FR)**

(72) Inventeur: **Lejemble, Philippe, 23 rue des Cédres, F-31400 Toulouse (FR)**
Inventeur: **Gaset, Antoine, 75 allée de Brienne, F-31000 Toulouse (FR)**
Inventeur: **Kalck, Philippe, Lapradine Tolosane no 4, F-31320 Auzeville (FR)**
Inventeur: **Merle, Georges, Lieu-dit "Marcetou" Saint-Ferreol, F-47240 Bon Encontre (FR)**
Inventeur: **Molinier, Bernard, Lotissement Carcy Rue de Mamène, F-47000 Agen (FR)**

(74) Mandataire: **Barre, Philippe, Cabinet Barre, Gatti, Laforgue 95, rue des Amidonniers, F-31069 Toulouse Cedex (FR)**

## Description

L'invention concerne un procédé de décarbonylation du furfural en vue d'obtenir du furanne; elle s'étend à un catalyseur perfectionné de décarbonylation.

La décarbonylation du furfural peut être réalisée en chauffant celui-ci en présence de deux grands types de catalyseurs de nature différente:

– soit des oxydes ou mélanges d'oxydes qui exigent que le milieu réactionnel soit porté à très haute température et présentent le défaut de se désactiver très rapidement, dans des conditions rendant leur utilisation incompatible avec des applications industrielles,

– soit les métaux nobles du groupe VIII qui sont plus favorables à la conversion et parmi ceux-ci, tout particulièrement le palladium qui s'est avéré le plus propice.

La présente invention vise un procédé de décarbonylation du dernier type, utilisant un catalyseur à base de palladium.

La décarbonylation du furfural, en présence de palladium, a été réalisée pour la première fois en 1952 (Eschinazi, Bull. Soc. Chim. Fr, 1952, 967–969) mais présente le double défaut, consistant, d'une part en une activité assez faible, c'est-à-dire une productivité relativement réduite en furanne (rapportée à un gramme de palladium), d'autre part en une désactivation du catalyseur relativement rapide.

Plusieurs études ont été menées consécutivement aux travaux d'Eschinazi pour tenter d'obtenir à la fois une meilleure productivité et une désactivation plus lente.

Une de ces études (brevet US n° 3 223 714) a consisté à élever la température jusqu'à une valeur de 300°C et à effectuer la réaction en présence d'hydrogène. Ce procédé améliore très notablement la productivité mais non la rapidité de désactivation du catalyseur; de plus, il est de mise en œuvre beaucoup plus complexe et conduit à des consommations énergétiques non négligeables, puisqu'il s'effectue à haute température sous atmosphère spécifique.

D'autres études ont donné des résultats plus remarquables, notamment sur le plan de la lenteur de désactivation (brevet US n° 3 007 941 et n° 3 257 417); elles ont conduit à ajouter au catalyseur à base de palladium, un sel de métal alcalin ou alcalino-terreux tel que de l'acétate de calcium, ce qui a permis d'améliorer très sensiblement à la fois la productivité et la durée de vie du catalyseur; dans les procédés conus de ce type, la réaction est réalisée à une température comprise entre 200 et 230°C: à ces températures, l'on obtient, au terme de la réaction, un mélange de furfural et de furanne, et le furanne pur est séparé de ce mélange par distillation.

Toutefois, dans les applications industrielles, il s'avère que la régénération du catalyseur présente une influence importante sur le coût du procédé et donc sur le prix de revient du furanne fabriqué; la préoccupation des industriels demeure donc toujours centrée sur le problème d'augmentation de la durée de vie du catalyseur et accessoirement, bien entendu, sur celui d'un accroissement de la productivité; toute amélioration de ces facteurs a un impact très sensible sur le prix du furanne fabriqué mais il n'a pas été possible depuis les études évoquées plus haut (1966) de mettre au point des perfectionnement permettant d'obtenir une telle amélioration.

La présente invention se propose d'indiquer un procédé amélioré permettant d'augmenter, de façon significative, à la fois, la durée de vie du catalyseur et la productivité de la réaction.

Un autre objectif de l'invention est d'abaisser la température de la réaction de décarbonylation, afin d'autoriser des économies notables sur le plan énergétique.

Un autre objectif, également important de l'invention, est de permettre de réaliser la réaction dans une plage de température adaptée pour obtenir, en une seule étape, le furanne pur, avec le produit secondaire produit par la réaction (monoxyde de carbone), sans avoir à effectuer une étape séparée de distillation pour éliminer le furfural.

Le procédé de décarbonylation visé par la présente invention est du type consistant à chauffer le furfural en présence d'un catalyseur à base de palladium déposé sur un support et à distiller le furanne formé; conformément a la présente invention, l'on réalise préalablement une irradiation du catalyseur à base de palladium, au moyen d'un rayonnement électromagnétique de longueur d'onde approximativement comprise entre 180 et 400 nanomètres. On a pu constater que le résultat optimum était obtenu lorsque le catalyseur était préalablement irradié au moyen d'un rayonnement ultraviolet de longueur d'onde comprise entre 230 et 270 nanomètres et lorsque cette irradiation était effectuée pendant une durée approximativement comprise entre 30 et 90 minutes.

Comme le montreront les exemples décrits plus loin, l'utilisation d'un tel catalyseur irradié augmente très sensiblement la durée de vie du catalyseur, c'est-à-dire l'activité de celui-ci au terme d'un certain temps de réaction. De même, la productivité initiale du catalyseur se trouve significativement augmentée.

Comme cela est connu en soi, on utilise de préférence dans le procédé de l'invention un catalyseur à base de palladium mélangé à un sel de métal alcalin ou alcalinoterreux; dans ce cas, selon une autre caractéristique du procédé de l'invention, l'irradiation du catalyseur est réalisée sur le mélange, de façon à porter à la fois sur le palladium et sur le sel. On a pu constater que, dans ces conditions, la productivité et la durée de vie du catalyseur étaient très fortement améliorées, d'une part comme cela est connu en soi, par l'emploi du sel, d'autre part par l'irradiation du mélange sel-palladium, qui paraît déterminer un effet de synergie améliorant considérablement la durée de vie et la productivité du procédé.

Il est à noter qu'il est essentiel que l'irradiation soit réalisée sur le mélange de palldium et de sel et non sur le palladium seul; en effet, de façon inattendue, on a remarqué que, lorsque la réaction était réalisée en présence de sel, l'irradiation du palladium seul ne donnait qu'une amélioration négligeable ou même nulle; dans certains cas, il s'est même avéré que l'utilisation du sel non irradié avec du palladium déjà irradié donnait des résultats moins bons que l'utilisation du sel et du palladium sans irradiation.

En conséquence, sur le plan industriel, il sera extrêmement avantageux d'utiliser un catalyseur à base de palladium mélangé à un sel de métal alcalin ou alcalino-terreux, ledit mélange ayant été préalablement irradié comme défini ci-dessus; le sel utilisé sera de préférence du carbonate de potassium qui paraît fournir les meilleurs résultats.

Par ailleurs, on a également pu constater que la réaction conforme à l'invention en présence du catalyseur irradié présente le meilleur rendement à une température approximativement comprise entre 140 et 180°; dans ces conditions, le furfural et le catalyseur sont de préférence chauffés sensiblement à 162°C, à pression atmosphérique, de façon à travailler au reflux du furfural (dont la température d'ébullition est de 162°C à pression atmosphérique).

Ainsi, dans une seule étape, on réalise la décarbonylation du furfural et la distillation du furanne formé dont les vapeurs peuvent être directement condensées dans un récepteur (température d'ébullition du furanne: 32°C).

Par ailleurs, on a pu remarquer que les conditions suivantes de mise en œuvre fournissent les meilleurs résultats:

– la proportion pondérale de sel de métal alcalin ou alcalino-terreux mélangé au palladium est approximativement comprise entre 10 et 70 grammes de sel par gramme de palladium,

– le support du catalyseur est du charbon, en proportion pondérale approximativement comprise entre 1 et 10 grammes de palladium par 100 grammes de charbon,

– le charbon utilisé comme support de catalyseur présente une surface spécifique approximativement comprise entre 500 et 1 500 m$^2$ par gramme,

– le catalyseur irradié est mis en présence du furfural dans une proportion pondérale approximativement comprise entre $1.10^3$ et $7.10^3$ g de furfural par g de palladium,

– le catalyseur est utilisé pour réaliser la réaction immédiatement après l'irradiation ou dans les heures qui suivent celle-ci,

– le démarrage de la réaction est assuré sous atmosphère inerte.

L'invention s'étend à l'utilisation d'un catalyseur de décarbonylation caractérisé en ce qu'il est à base de palladium irradié au moyen d'un rayonnement ultraviolet et plus spécialement d'un mélange irradié de palladium et de sel de métal alcalin ou alcalino-terreux.

Il est à noter que, dans un autre type de réaction, (hydrogénation d'un cyclohexène pour obtenir un cyclohexane: CHEMICAL ABSTRACTS – Vol. 95 n$_o$ 5 KK. KUZEMBAEV et al.) il est utilisé un catalyseur à base de palladium qui est soumis préalablement à un rayonnement bêta ou gamma.

Toutefois, il s'agit d'un procédé sans rapport avec celui de l'invention, puisque:

(1) il porte sur un procédé d'hydrogénation (et non de décarbonylation),

(2) il s'applique sur un corps de nature très différente de celle du corps visé par l'invention (cyclohexène et non furfural),

(3) il utilise une irradiation de nature très différente de celle utilisée dans l'invention (rayonnements gamma ou bêta: la rayonnement bêta est une émission de particules et le rayonnement gamma présente des longueurs d'ondes de l'ordre de cent mille fois plus faible que les rayonnements ultraviolets ce qui rend leur propriété totalement indépendante).

En conséquence, ces seules considérations suffiraient à écarter cette «technique KUZEMBAEV» qui ne pourrait en aucun cas rendre évidente l'invention du demandeur.

Mais celle-ci peut également être écartée pour d'autres raisons péremptoires.

En effet, les inventeurs ont effectué une décarbonilation du furfural en présence d'un catalyseur irradié, non plus au moyen d'un rayonnement ultraviolet mais au moyen d'un rayonnement bêta ou gamma, comme l'enseigne KUZEMBAEV dans le cas de l'hydrogénation du cyclohexène. Ils se sont alors rendus compte que l'effet de cette irradiation est nul et, en tout cas, n'a pu être significativement décelé.

Par exemple, dans le cas d'un catalyseur formé par du palladium sur charbon, le rendement horaire (nombre de moles de furane obtenu par mole de palladium) a été de 76 au bout d'une heure avec un catalyseur non irradié et de 76 également avec un catalyseur irradié au moyen d'un rayonnement gamma de 30 kilorad pendant 15 heures.

En conséquence, non seulement les expérimentations de KUZEMBAEV ne rendent pas l'invention du demandeur évidente, mais encore, elle en détourne l'esprit de l'homme du métier, puisque le transfert des caractéristiques d'irradiation de KUZEMBAEV à la réaction de l'invention ne produit aucun résultat.

L'invention est illustrée par la description qui suit, qui présente plusieurs exemples de mise en œuvre du procédé.

Les exemples 1, 2, 3, 4 et 5 sont des exemples comparatifs destinés à montrer l'amélioration apportée par le procédé de l'invention par rapport aux procédés connus, soit en l'absence de sel (exemples 1 et 2), soit en présence de sel (exemples 3, 4 et 5), cependant que les exemples 6 à 10 présentent diverses conditions de mise en œuvre du procédé afin de mettre en évidence les conditions optimales, sur le plan du temps d'irradiation du catalyseur (exemple 6), de la longueur d'onde des rayonnements (exemple 7), de la densité énergétique des rayonnements (exemple 8).

## Exemple 1

Préparation de furanne en présence de palladium irradié.

Dans cet exemple, le catalyseur est constitué par un support en charbon de surface spécifique de 1 000 m² par gramme, sur lequel a été déposé du palladium en proportion pondérale égale à 5 grammes de palladium pour 100 grammes de charbon.

Ce catalyseur est placé sur un porte-échantillon soumis à un rayonnement ultraviolet délivré par une lampe d'ultraviolets de type classique; la longueur d'onde de l'irradiation est en l'exemple de 254 nanomètres; la densité énergétique au niveau du catalyseur est très approximativement de l'ordre de 0,5 watt par cm² et l'irradiation a été poursuivie pendant 1 heure.

Ce catalyseur irradié, se présentant sous la forme d'une poudre, a été utilisé immédiatement après son irradiation; il a été placé dans un réacteur de 250 ml surmonté d'une colonne de distillation de 40 cm de haut. Dans le réacteur ont été introduits 150 ml de furfural, le poids du palladium du catalyseur étant de 900 mg (soit 3800 grammes de furfural par gramme de palladium).

Le milieu réactionnel est chauffé à 162°C pendant 100 heures à la pression atmosphérique avec agitation mécanique du milieu au moyen de pales tournant à 270 tours par minute; au début de la réaction, le réacteur et la colonne de distillation sont placés sous atmosphère inerte d'azote qui est évacuée au fur et à mesure de la production de furanne et de monoxyde de carbone.

Les vapeurs de furanne produites au cours de la réaction sont condensées dans un récipient récepteur porté à une température égale à –30°C; le furanne obtenu est recueilli chaque heure, et du furfural en quantité correspondante est ajouté au milieu réactionnel pour maintenir sa concentration sensiblement à sa valeur initiale.

Le furanne obtenu a été analysé, d'une part au terme de la première heure afin de déterminer le rendement horaire initial, puis au terme de la centième heure afin de déterminer le rendement horaire au cours de la dernière heure de réaction.

Les analyses ont été effectuées par chromatographie en phase gazeuse sur colonne SE 30, le furanne étant caractérisé par son spectre $^1H$ RMN; ces résultats ont été ensuite confirmés par analyse élémentaire.

Le rendement horaire exprimé par le rapport du nombre de moles de furanne obtenu sur le nombre de moles de palladium a été de 98 au terme de la 1re heure.

Le rendement horaire au cours de la dernière heure a été de 34.

Des valeurs qui sont à comparer avec celles de l'exemple 2 montrent, d'une part, que la productivité initiale de la réaction a été améliorée d'environ 25%, d'autre part, que le rendement au cours de la centième heure est supérieur à celui obtenu dans le cas du catalyseur non irradié (environ 10%): le catalyseur irradié peut être utilisé sans régénération avec un rendement satisfaisant pendant un temps beaucoup plus long.

La même expérience que ci-dessus a été reconduite dans les mêmes conditions jusqu'à obtenir un rendement du mêmes ordre que celui obtenu au terme de l'essai de l'exemple 2 (30). Il a fallu environ 200 heures pour que le rendement s'abaisse au niveau de celui de l'exemple 2, de sorte que, à rendement final égal, la durée de vie du catalyseur a été multipliée par un facteur égal à 2.

## Exemple 2

Fabrication de furanne en présence de palladium non irradié.

A titre comparatif, des expérimentations analogues à celles de l'exemple 1 ont été menées avec un catalyseur de nature identique, mais non soumis à l'opération d'irradiation.

On constate que le rendement horaire au cours de la première heure est de 76 et que le rendement horaire au terme de la centième heure est de 30.

## Exemple 3

Fabrication de furanne en présence d'un mélange irradié de palladium et de carbonate de potassium.

Dans cet exemple, le catalyseur est constitué par du charbon en poudre sur lequel a été déposé du palladium et auquel a été mélangé un promoteur constitué par du carbonate de potassium également en poudre. La proportion pondérale de palladium par rapport au charbon est de 5 grammes de palladium pour 100 grammes de charbon, cependant que la proportion pondérale de carbonate de potassium est de 20 grammes de carbonate de potassium par gramme de palladium. Ce mélange catalyseur-promoteur est irradié dans les mêmes conditions que celles définies à l'exemple 1.

Il est disposé dans un réacteur du même type et mélangé à du furfural à raison de 3 800 grammes de furfural par gramme de palladium. Après démarrage sous atmosphère inerte, le milieu réactionnel est chauffé à 162°C et on procède ensuite sous les mêmes conditions opératoires que les exemples précédents.

Le rendement horaire au cours de la première heure est de 176, cependant que ce rendement au cours de la centième heure est de 74. On peut constater, d'une part, uen nette amélioration de la productivité, d'autre part, une désactivation beaucoup plus lente du catalyseur qui garde au bout de la centième heure une activité exceptionnelle.

## Exemple 4

Préparation de furanne en présence d'un mélange catalyseur non irradié de sel et de palladium.

Les mêmes expérimentations que celles de l'exemple 3 sont effectuées sans soumettre le mélange catalyseur aux rayonnements ultraviolets.

Le rendement horaire au terme de la première heure est de 155, tandis que le rendement horaire au terme de la centième heure est de 56.

On constate donc que le mélange catalyseur irradié utilisé à l'exemple 3 possède une meilleure productivité initiale et subit une désactivation beaucoup plus lente.

Exemple 5

Préparation de furanne en présence d'un mélange de palladium et de carbonate de potassium dans lequel seul le palladium est irradié.

Dans cet exemple, le palladium déposé sur charbon dans les mêmes conditions qu'à l'exemple 1 est irradié de la même façon qu'à cet exemple.

Après irradiation, du carbonate de potassium est ajouté au corps précédent dans les mêmes proportions que celles définies à l'exemple 3. La réaction menée dans les mêmes conditions que précédemment avec un tel catalyseur pendant 5 heures, donne un rendement horaire au cours de la première heure de 136. On constate donc que, lorsqu'on utilise le palladium mélangé à un sel, il est essentiel d'irradier le mélange des deux, sous peine de pénaliser la productivité initiale de la réaction. Compte tenu de ce résultat, cette réaction n'a pas été poursuivie au-delà de 5 heures.

Exemple 6

Influence du temps d'irradiation.

La même expérimentation que celle effectuée à l'exemple 3 est effectuée en faisant varier le temps d'irradiation du mélange catalyseur, la réaction étant effectuée pendant 5 heures de façon à déterminer le rendement initial.

Pour un temps d'irradiation de 45 minutes, le rendement initial a été de 147; pour un temps d'irradiation de 75 minutes, ce rendement a été de 160; pour un temps d'irradiation de 90 minutes, il a été de 148.

On constate donc que l'optimum est obtenu dans les conditions d'irradiation de l'exemple 3; toutefois le rendement reste satisfaisant dans une plage de 30 à 90 minutes d'irradiation.

Exemple 7

Influence de la longueur d'ondes d'irradiation.

On effectue dans les mêmes conditions que celles de l'exemple 3 la réaction avec un mélange catalyseur irradié par un rayonnement de longueur d'onde différente, égale à 366 nanomètres. Le rendement initial est nettement inférieur à celui de l'exemple 3 et dans ce cas est de l'ordre de 92.

Exemple 8

Influence de la densité énergétique.

Les mêmes expérimentations que celles de l'exemple 1 sont réalisées avec des lampes de puissance différente, l'une donnant une densité énergétique sur le catalyseur de l'ordre de 0,5 watt par $cm^2$, l'autre une densité énergétique de 40 watts par $cm^2$. On constate que les rendements ne sont pas significativement différents; la plage des densités énergétiques applicables paraît très large et pourra par exemple être ajustée entre 0,1 et 100 watts/$cm^2$.

Exemple 9

Influence de la proportion de sel dans le catalyseur.

Les mêmes expérimentations que celles de l'exemple 3 ont été effectuées en faisant varier dans le catalyseur la proportion de sel par rapport au palladium. Dans une première expérimentation, on a utilisé un catalyseur dont la proportion de sel était de 15 grammes de sel par gramme de palladium; dans une deuxième expérimentation, cette proportion était portée à 18 grammes et dans une troisième expérimentation, à 25 grammes.

Les rendements horaires initiaux sont respectivement pour ces trois expérimentations de 97, 148 et 133.

On constate donc que la proportion de l'exemple 3 est la meilleure.

Exemple 10

Influence de la proportion de catalyseur par rapport au furfural.

Les mêmes expérimentations que celles de l'exemple 3 ont été reproduites en faisant varier la proportion de furfural par rapport au catalyseur. On trouvera ci-dessous les proportions essayées et les rendements initiaux correspondant à celles-ci:

– 4 150 g de furfural pour 1 g de palladium; rendement: 119,

– 3 500 g de furfural pour 1 g de palladium; rendement: 100.

On constate qu'ici aussi, les conditions de l'exemple 3 sont optimales.

**Revendications**

1. Procédé de décarbonylation du furfural en vue d'obtenir du furanne, ce procédé étant du type consistant à chauffer le furfural en présence d'un catalyseur à base de palladium déposé sur un support et à distiller le furanne formé, et étant caractérisé en ce que l'on réalise préalablement une irradiation, du catalyseur au moyen d'un rayonnement ultraviolet.

2. Procédé selon la revendication 1, caractérisé en ce que le catalyseur est préalablement irradié au moyen d'un rayonnement ultraviolet de longueur d'onde comprise entre 230 et 270 nanomètres.

3. Procédé selon la revendication 2, caractérisé en ce que le catalyseur est irradié sous une densité énergétique de rayonnement comprise entre 0,1 et 100 watts/$cm^2$ pendant une durée comprise entre 30 et 90 minutes.

4. Procédé selon l'une des revendications 1, 2 ou 3, caractérisé en ce que l'on utilise le catalyseur dans les heures qui suivent l'irradiation.

5. Procédé selon l'une des revendications 1, 2, 3 ou 4, dans lequel on utilise un catalyseur à base de palladium mélangé à un sel de métal alcalin ou alcalino-terreux, caractérisé en ce que l'irradiation du catalyseur est réalisée sur le mélange.

6. Procédé selon la revendication 5 dans lequel le sel mélangé au catalyseur et irradié avec celui-ci est du carbonate de potassium.

7. Procédé selon l'une des revendications 5 ou 6, caractérisé en ce que la proportion pondérale de sel mélangé au palladium est comprise entre 10 et 70 g de sel par g de palladium.

8. Procédé selon l'une des revendications 1, 2, 3, 4, 5, 6 ou 7, dans lequel le support de catalyseur est du charbon, en proportion pondérale comprise entre 1 et 10 g de palladium pour 100 g de charbon.

9. Procédé selon la revendication 8, caractérisé en ce que l'on utilise comme support de catalyseur, du charbon ayant une surface spécifique comprise entre 500 et 1 500 $m^2$/g.

10. Procédé selon l'une des revendications précédentes, dans lequel le catalyseur irradié est mis en présence du furfural dans une proportion pondérale comprise entre $1.10^3$ et $7.10^3$ g de furfural par g de palladium.

11. Procédé selon l'une des revendications précédentes, caractérisé en ce que le furfural et le catalyseur sont chauffés à une température comprise entre 140° et 180°C.

12. Procédé selon la revendication 11, caractérisé en ce que le furfural et le catalyseur sont chauffés à 162°C, à pression atmosphérique.

13. Procédé selon l'une des revendications précédentes, caractérisé en ce que l'on assure le démarrage de la réaction sous atmosphère inerte.

14. Utilisation d'un catalyseur à base de palladium irradié au moyen d'un rayonnement ultraviolet dans un procédé de décarbonylation du furfural.

15. Utilisation selon la revendication 14, d'un catalyseur à base d'un mélange irradié de palladium et de sel de métal alcalin ou alcalino-terreux.

**Patentansprüche**

1. Verfahren zum Decarbonylieren des Furfurals zwecks Herstellung des Furans, wobei dieses Verfahren darin besteht, dass man das Furfural in Gegenwart eines auf einem Träger angeordneten Katalysators auf Palladium-Basis erhitzt und das gebildete Furan destilliert, und dadurch gekennzeichnet ist, dass man zuerst eine Bestrahlung des Katalysators mittels einer ultravioletten Strahlung vornimmt.

2. Verfahren nach Anspruch 1, dadurch gekennzeichnet, dass der Katalysator zuerst mittels einer ultravioletten Strahlung mit einer zwischen 230 und 270 Nanometern liegenden Wellenlänge bestrahlt wird.

3. Verfahren nach Anspruch 2, dadurch gekennzeichnet, dass der Katalysator in einer zwischen 0,1 und 100 Watt/$cm^2$ liegenden Strahlungsenergie-Dichte während einer zwischen 30 und 90 Minuten liegenden Zeitdauer bestrahlt wird.

4. Verfahren nach einem der Ansprüche 1, 2 oder 3, dadurch gekennzeichnet, dass man den Katalysator während der auf die Bestrahlung folgenden Stunden verwendet.

5. Verfahren nach einem der Ansprüche 1, 2, 3 oder 4, bei welchem man einen Katalysator auf Palladium-Basis in Mischung mit einem Alkalioder Erdalkalisalz verwendet, dadurch gekennzeichnet, dass die Bestrahlung des Katalysators auf die Mischung ausgeübt wird.

6. Verfahren nach Anspruch 5, bei welchem das mit dem Katalysator gemischte und mit diesem bestrahlte Salz Kaliumcarbonat ist.

7. Verfahren nach einem der Ansprüche 5 oder 6, dadurch gekennzeichnet, dass der Gewichtsanteil von mit dem Palladium vermischten Salz zwischen 10 und 70 g Salz pro g nach einem der Ansprüche 1, 2, 3, 4, 5, 6 oder 7, bei welchem es sich bei dem Katalysator-Träger um Kohle in einem Gewichtsanteil zwischen 1 und 10 g Palladium auf 100 g Kohle handelt.

9. Verfahren anch Anspruch 8, dadurch gekennzeichnet, dass man als Katalysator-Träger Kohle mit einer spezifischen Oberrfläche zwischen 500 und 1 500 $m^2$/g verwendet.

10. Verfahren nach einem der vorangehenden Ansprüche, bei welchem der bestrahlte Katalysator mit dem Furfural in einem Gewichtsverhältnis zwischen $1.10^3$ und $7.10^3$ g Furfural pro g Palladium zusammengebracht wird.

11. Verfahren nach einem der vorangehenden Ansprüche, dadurch gekennzeichnet, dass das Furfural und der Katalysator auf eine zwischen 140° und 180°C liegende Temperatur erhitzt werden.

12. Verfahren nach Anspruch 11, dadurch gekennzeichnet, dass das Furfural und der Katalysator auf 162°C bei Atmosphärendruck erhitzt werden.

13. Verfahren nach einem der vorangehenden Ansprüche, dadurch gekennzeichnet, dass man die Reaktion unter inaktiver Atmosphäre in Gang setzt.

14. Verwendung eines mittels einer ultravioletten Strahlung bestrahlten Katalysators auf Palladium-Basis bei einem Verfahren zum Decarbonylieren von Furfural.

15. Verwendung nach Anspruch 14 eines Katalysators auf Basis eines bestrahlten Gemischs von Palladium und Alkali- oder Erdalkalisalz.

**Claims**

1. Process for the decarbonylation of furfural with a view to producing furan, this process consisting in heating furfural in the presence of a palladium-based catalyst, placed on a carrier, and in distilling the resulting furan, and being characterised in that, at first, irradiation of the catalyst is carried out by means of ultraviolet radiation.

2. Process according to Claim 1, characterised in that the catalyst is irradiated, at first, by means of ultraviolet radiation having a wavelength of between 230 and 270 nanometres.

3. Process according to Claim 2, characterised in that the catalyst is irradiated in a density of radiating energy of between 0.1 and 100 watts/cm² for a period of between 30 and 90 minutes.

4. Process according to one of the Claims 1, 2 or 3, characterised in that the catalyst is used during the hours following irradiation.

5. Process according to one of the Claims 1, 2, 3 or 4, in which a palladium-based catalyst, mixed with an alkali metal salt or alkaline earth metal salt, is used, characterised in that the irradiation of the catalyst is carried out on the mixture.

6. Process according to Claim 5, in which the salt, mixed with the catalyst and irradiated with the latter, is potassium carbonate.

7. Process according to either Claim 5 or 6, characterised in that the proportion by weight of salt mixed with the palladium lies between 10 and 70 grams of salt per gram of palladium.

8. Process according to one of the Claims 1, 2, 3, 4, 5, 6 or 7, in which the catalyst carrier is charcoal, in a proportion by weight of between 1 and 10 grams of palladium to 100 grams of charcoal.

9. Process according to Claim 8, characterised in that the catalyst carrier used is charcoal having a specific surface of between 500 and 1,500 m²/g.

10. Process according to one of the preceding claims, in which the irradiated catalyst is brought into contact with the furfural in a proportion by weight of between $1.10^3$ and $7.10^3$ grams of furfural per gram of palladium.

11. Process according to one of the preceding claims, characterised in that the furfural and the catalyst are heated to a temperature of between 140° and 180°C.

12. Process according to Claim 11, characterised in that the furfural and the catalyst are heated to 162°C at atmospheric pressure.

13. Process according to one of the preceding claims, characterised in that the reaction is started in an inert atmosphere.

14. Use of a palladium-based catalyst, irradiated by means of ultraviolet radiation, in a process for the decarbonylation of furfural.

15. Use, according to Claim 14, of a catalyst based on an irradiated mixture of palladium and alkali metal salt or alkaline earth metal salt.